# EUROPEAN PATENT APPLICATION

(11) **EP 1 798 236 A1**
(43) Date of publication of application: **20.06.2007**
(21) Application number: 05112017.8
(22) Date of filing: 13.12.2005
(51) Int. Cl.: C07F 9/58, A61P 35/00

(54) **Process for the preparation of 3-pyridyl-1-hydroxyethylidene-1,1- biphosphonic acid and hydrated forms thereof**

(71) Applicant: EOS Eczacibasi Ozgun Kimyasal Urunler Sanayi Ve Ti Caret A.S., 80640 Istanbul (TR)
(72) Inventor: Karliga, Bekir, EOS Eczacibasi Ozgun Kimyasal, 80640 Istanbul (TR); Keskin, Hulya, EOS Eczacibasi Ozgun, 80640 Istanbul (TR); Adiyaman, Mustafa, EOS Eczacibasi Ozgun, 80640 Istanbul (TR)
(74) Representative: Sevinç, Erkan

(57) **Abstract**

A process for the preparation of 3-pyridyl-1-hydroxyethylidene-1,1-biphosphonic acid or pharmaceutically acceptable salts thereof are disclosed. The method comprises reacting 3-pyridyl acetic acid with phosphorous acid and phosphorous trichloride where the reaction is carried out in the presence of an emulsifying agent. The emulsifying agent can be selected from aralkyl or alkyl ethoxylates or derivates thereof having the general formula of R-X-O-(CH₂CH₂O)ₙ-H, or triglycerides having the general formula of (RCO₂CH₂)₂CHOCOR or oils such as plant and animal oils containing such triglycerides, or alkanes having the general formula CₘH₂ₘ₊₂. The reaction is followed by recovering said 3-pyridyl-1-hydroxyethylidene-1,1-bisphosphonic acid or metal salts thereof by the hydrolysis reaction of phosphorous intermediates.

## Description

### Technical Field of the Invention

The present invention relates to a method for preparation of 3-pyridyl-1-hydroxyethylidene-1,1-bisphosphonic acid and its pharmaceutically acceptable salts, in anhydrous or hydrated forms. The core underlying the present invention relates to using emulsifying agents in the phosphonylation reactions and to the anhydrous or hydrated forms of products obtained through such reaction.

### Background of the Invention

Biphosphonic acids have been widely proposed for the treatment or prevention of diseases including bone resorption, hypercalcemia of malignancy, Paget's disease and osteoporosis. 3-pyridyl-1-hydroxyethylidene-1, 1-bisphosphonic acid, as referred risedronic acid is one of the useful active pharmaceutical ingredients for treatment of such diseases. Risedronic acid posses chemically unique compound structure as compared with the other biphosphonates which is believed to reduce the likelihood of gastro-intestinal side effects. It is also more potent in blocking the dissolution of bone rather than etidronate and alendronate.

The present invention discloses a process for producing risedronic acid, appropriate pharmaceutically acceptable salts and hydrated forms. Following a conventional pathway as in the prior art, phosphonylation reaction for synthesizing risedronate is carried out with the reagents 3-pyridyl acetic acid, phosphorus acid and phosphorus trichloride. Emulsifying agents employed in the reaction medium succeed the objects of the invention that are disclosed in detail below. Preferable emulsifying agents are mainly aralkyl or alkyl ethoxylates, long chain alkanes such as paraffins and triglycerides which are highly available in the plant or animal oils.

Biphosphonates employing a pyridyl ring in the molecule chain are disclosed with a wide range of biphosphonic molecules in EP 0186405 wherein objects of the disclosure appears to be the provision of useful pharmaceutical compositions containing geminal diphosphonates for treatment of diseases such as abnormal calcium and phosphate metabolism. In one embodiment of the disclosure 2-(2-pyridyl)-1-hydroxyethane-1,1-diphosphonic acid which is an analogue of risedronic acid is mentioned in the description. Overall process does not have a stable temperature range and too complex to carry out which makes the temperature and other parameters of the process uncontrollable.

WO 03/093282 exemplifies a similar process for producing risedronic acid wherein 3-pyridyl acetic acid is used as the starting reagent. However, use of toluene in the reaction medium makes the process unsafe as this solvent is highly flammable. Nonhomogeneous medium having more than one liquid phase is another drawback of this method which readily results lower yield and lower quality of product.

The patent application US 2004/043967 (Lidor-Hadas et al.) discloses a process which differs from the classical pathway of the art with respect to the diluents employed in the reaction medium. Such diluents are reported to be an aromatic hydrocarbon or a silicon oil. In one embodiment of the disclosure aromatic hydrocarbons such as toluene are used as diluent agents in the reaction medium. One skilled in the art may readily recognize that the process of this application involves safety related problems as well as the problems related with the product impurity.

In a well known method of producing risedronic acid described in Journal of Organic Chemistry Vol. 60, No 25, pp. 8310-8312 (1995) a reaction of 3-pyridylacetic acid with phosphorous acid and phosphorus trichloride is explained in detail. In accordance with the disclosure of this article, the major technical problem is the solid state hardly mixed reaction medium which makes large scale production difficult and even impossible. This problem is tried to be overcome by use of methanesulphonic acid, however environmental drawbacks of methanesulphonic acid makes this process industrially inapplicable. Furthermore, use of methanesulfonic acid in the presence of phosphorous trichloride arises a serious safety problem since methanesulfonic acid reacts with phosphorous trichloride, under adiabatic conditions, the reaction becomes self-heating at 85 °C and an uncontrollable exotherm occurs at reaction temperatures above 140 °C.

EP 1243592 discloses a process for the preparation of risedronic acid by reacting 3-pyridylacetic acid with phosphorous acid and phosphorus trichloride in a solvent, particularly in chlorobenzene or fluorobenzene. Although the procedures of product purification are significantly simplified and energy consumption of the process is reduced, use of such volatile solvents like chlorobenzene and fluorobenzene restricts the temperature range of the process up to reflux temperature unless a convenient pressure to the reaction medium is not applied. Furthermore, relatively high amount of gaseous chlorine emitted during the phosphonylation reaction causes serious health risks.

Numerous methods for producing hydrated forms and various crystal structures of risedronic acid are proposed in the prior art.

WO 01/56983 describes a process for selective crystallization of risedronic acid sodium as the hemipentahydrate or monohydrate through control of the crystallization temperature and rate of crystallization. The process is conducted in the presence of a solvent selected from the group of alcohols, esters, ethers, ketones, amides, and nitriles. WO 2004/037252 discloses a similar process for crystallizing risedronic acid and the product obtained thereby. Preferred solvent used in this procedure is an alcohol, particularly 2-propanol.

WO 02/090367 discloses a process for producing 4-amino-1-hydroxybutylidene-1,1-biphosphonic acid wherein the reaction of 4-aminobutyric acid with phosphorous acid and phosphorous trichloride is carried out in the presence of aralkyl or alkyl ethoxylates or triglycerides which reflects similarity with the present invention in the sense of emulsifying agents. It has been found that use of such emulsifying agents for the preparation of risedronic acid surprisingly resulted a yield increase of 3 to 5 %, which is a very important manufacturing success in the pharmaceutical industry. In addition, the present invention showed that a considerable impurity decrease has been accomplished compared to the process of producing alendronic acid.

The problems relating to safety and mixing of reaction medium in an easily operable and stable one single process still remains to be solved simultaneously for the risedronic acid production. Emulsifying agents of the present invention provide not only safe operation and homogeneous mixing, but also provide easy operation in a wide range of temperature values, stable yield and lower impurity in the final product.

### Summary of the Invention

The present invention discloses a process for the preparation of 3-pyridyl-1-hydroxyethylidene-1,1-bisphosphonic acid and its salts, in anhydrous or hydrated forms wherein the reaction of 3-pyridyl acetic acid with phosphorus acid and phosphorus trichloride is carried out in the presence of aralkyl or alkyl ethoxylates or triglycerides or alkanes.

The ethoxylates used in the reaction medium are selected from the group of compounds having the general formula of R-X-O-(CH₂CH₂O)ₙ-H. Plant oils or their derivatives are the convenient triglyceride sources which may well be tailored as the emulsifying agents for the reaction conditions. Triglycerides having the general formula of (RCO₂CH₂)₂CHOCOR shall be of particular interest of the present invention. Alkanes having the general simple formula CₙH₂ₙ₊₂ are another embodiment of the present invention where the paraffins having carbon content greater that 20 are used as suitable alkane sources.

Risedronic acid sodium is the preferable final product of the present invention which may well be in anhydrous or hydrated forms, particularly in monohydrate or hemipentahydrate forms depending on the water content allowed during the operation of the process. The problems faced in the art have been solved through use of aforementioned emulsifying agents that underlie the principle idea of the present invention.

### Objects of the Invention

One of the objects of the present invention is to provide a safe process for the production of risedronic acid, in which process conventional flammable or explosive organic or inorganic solvents do not exist.

Another object of the present invention is to provide a process in which homogeneous mixing is achieved, thereby a stable yield and reduced impurity values are provided.

Another object of the present invention is to provide a simple and easily operable process, which does not comprise varying temperature values or complex operations.

Yet another object of the present invention is to provide a process which employs effectively reusable emulsifiers that enable easy recovery of the product after phosphonylation reaction.

### Detailed Description of the Invention

Risedronic acid is traditionally prepared by the reaction of 3-pyridyl acetic acid with phosphorus acid and phosphorus trichloride in the presence of organic solvents such as methanesulphonic acid, chlorobenzene, toluene and various alcohols and ketones. 3-Pyridyl acetic acid is the preferable starting reagent of the phosphonylation reactions.

Emulsifying agents used in the phosphonylation reactions play the key role in the overall process giving many consequences in terms of yield, mixing, product quality, operation temperature etc. Emulsifying agents, employed in the reaction medium of the present invention are selected from the group consisting of aralkyl or alkyl ethoxylates, triglycerides and alkanes which succeed the objects of the present invention by eliminating the problems in the art.

Aralkyl or alky ethoxylates are hereby represented with the general formula R-X-O-(CH₂CH₂O)ₙ-H wherein R represents branched or non-branched alkyl or alkenyl groups, X represents phenyl or naphtyl or -CH₂- groups; n is an integer of 1 to 30. Number of carbons in said alkyl groups may vary 1 to 20 and in the alkenyl groups 2 to 20. The preferable ethoxylates used in the present invention are nonylphenol 4 mol, 6 mol, 10 mol ethoxylate and lauryl alcohol ethoxylate.

Triglycerides are hereby represented with the general formula (RCO₂CH₂)₂CHOCOR wherein R represents branched or non branched alkyl or alkenyl groups. Number of carbons in said alkyl groups may vary 1 to 20 and in the alkenyl groups 2 to 20. It is well known in the art that conventional plant oils contain high amount of triglycerides having various number of carbons. Such oils can contain one or more than one glyceride as a mixture. As an example sunflower oil, olive oil and corn oil contain the glycerides of oleic acid, lineloic acid, strearic acid, myristic acid, behenic acid and arachidic acid in various ratios. For instance linoleic glyceride fraction of corn oil may vary from 34 - 62 %. Thus, using such oils as triglyceride sources of the reaction medium provides the practical advantage of utilizing very common oil types such as corn oil, sunflower oil, olive oil which are highly available and inexpensive in the market. Evidently, other types of oils such as almond oil, babassu oil, borage oil, blackcurrant seed oil, canola oil, castor oil, coconut oil, cottonseed oil, evening primrose oil, grapeseed oil, groundnut oil, mustard seed oil, palm kernel oil, peanut oil, sesame oil, soybean oil, palm oil, and animal oils such as liver oil may be chosen as the emulsifying agent in the phosphonylation reaction. Interestingly, it has been found out during the development of the present process, that reactions carried out in the presence of aforementioned oils resulted slightly more yield compared to that obtained by using other emulsifying agents i.e. aralkyl or alkyl ethoxylates or alkanes. The reason is believed to be the phosphorous content of the oils.

In another embodiment of the present invention, basic alkanes having the general formula CₙH₂ₙ₊₂ are used as emulsifying agents in the reaction medium. Alkanes, such as paraffins having more than 20 carbons are in particular interest of the present invention.

One of the main objectives of the present invention is focusing on use of above defined emulsifiers or derivatives thereof in the phosphonylation reaction. Said emulsifier agents keep the reaction medium homogeneous during the agitation and enables the product easily separated right after the reactions. Furthermore, they can readily be reused in repeated reactions thereby providing cost efficiency.

3-Pyridyl acetic acid and the phosphorous acid are suspended in one or a combination of the above mentioned emulsifying agents and reacted with phosphorous trichloride at a temperature which is convenient to handle the reagents in liquid form. However, one skilled in the art would appreciate that phosphorous oxychloride or phosphorous pentachloride may also be used instead of phosphorous trichloride as a reagent of phosphonylation reaction. Said reaction can be carried out at the temperature range of 70 to 150 °C, preferably at 110 - 120 °C. After stirring for 4 hours water is added to the reaction mixture and an additional agitation is applied at the reflux temperature. Phosphorous intermediates are hydrolyzed after heating the medium to prescribed temperature. At this stage the process can be managed to obtain the final product in a metal salt form by adjusting the pH to a suitable value. According to the present invention sodium salt of the risedronic acid is the preferable product obtained at the end of the sequence of operations, however, one skilled in the art may readily recognize that salts of risedronic acid may be acid addition salts, in particular the hydrochloride, and any pharmaceutically-acceptable, non-toxic organic or inorganic acid salt. In addition, salts formed with the phosphonic acid group may be obtained, including, but not limited to alkali metal salts (K, Na) and alkaline earth metal salts (Ca, Mg). NaOH is added to the reaction medium and pH is adjusted to 4.4 - 5.0, preferably to 4.7 for obtaining risedronate sodium. This stage is followed by a further agitation and precipitation of the product with addition of acetone.

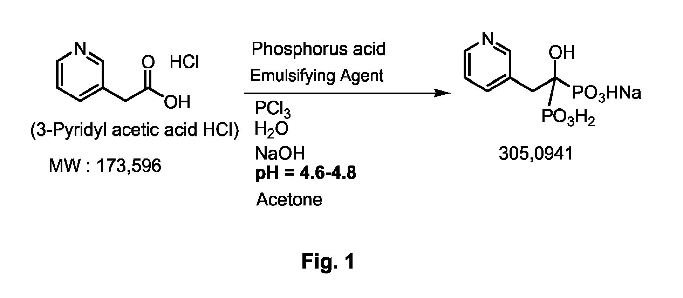

Obtaining the risedronic acid in anhydrous or hydrated forms are adjusted by a drying operation which absolutely affects the water content. The final product obtained at the end of the reactions can be adjusted to obtain the product in anhydrous form as well as in monohydrate or hemipentahydrate forms depending on the desired content of water. Water content of the product is 5-6 % for monohydrate form and 12-13 % for hemipentahydrate form. The reaction of the present invention is simply illustrated in Fig. 1.

As it may readily be seen from the combination of the information and Fig. 1, NaOH is used for obtaining the Na salt of risedronic acid and acetone is used for separating the final product from reaction mixture.

The following examples are given solely to illustrate the present invention without limiting the same in any way.

### Example 1

### Preparation of 3-pyridyl-1-hydroxyethylidene-1, 1-bisphosphonic acid monosodium salt in the presence of sunflower oil

1700 g of 3-Pyridyl acetic acid.HCl and 2000 g of phosphorus acid are charged through 25 L of sunflower oil and suspended in the same. Temperature is adjusted to 90 °C at which all components of the reaction medium are provided in liquid form. A NaOH trap is mounted to the system to catch the possible HCl emition. At this temperature 4700 g of phosphorous trichloride is added dropwise. After completion of said addition, temperature is adjusted to 115°C and reaction medium is stirred for 4 hours. Then reaction medium is allowed to cool down until 4 - 5 °C and 31 L of water is slowly added to the reaction mixture. The mixture is stirred for 4 hours at reflux temperature (110 °C) where the phosphorous intermediates are hydrolyzed and phases are separated thereafter. Water phase is filtered on celit and allowed to cool down until room temperature. The mixture at this temperature is treated with dropwise 50% NaOH solution (5.6 L) after which the pH is adjusted to 4.7. This mixture is stirred for 12 hours for obtaining the so formed white crystals in precipitated form. Cake of the crystalline precipitate is dissolved in 30 L of water and then by addition acetone (70L) product is re-precipitated. The yield of the overall process is 28 %. The analysis confirmed the identity of the product and the absence of impurities.

³¹ P-NMR, ¹H-NMR and ¹³C-NMR spectra were obtained in D₂O on a Varian Mercury 300 MHz instrument.

³¹P-NMR(D₂O): 17.34 (s) ;
¹H-NMR(D₂O): 3.26 (t, 2H), 7.72 (t, 1H), 8.37 (m, 2H), 8.54 (t, 1H)
¹³C-NMR(D₂O):149.04, 142.67, 138.62, 126.18, 36.2

### Example 2

### Preparation of 3-pyridyl-1-hydroxyethylidene-1,1-bisphosphonic acid monosodium salt in the presence of recovered sunflower oil

The procedure of Example I is repeated except recovered sunflower oil is used as the emulsifying agent instead of non-used sunflower oil. The analysis confirmed the identity of the product and the absence of impurities. Overall yield of 27.5 % is obtained.

### Example 3

### Preparation of 3-pyridyl-1-hydroxyethylidene-1, 1-bisphosphonic acid monosodium salt in the presence of olive oil

The procedure of Example I is repeated except olive oil is used as the emulsifying agent instead of sunflower oil. The analysis confirmed the identity of the product and the absence of impurities. Overall yield of 27.0 % is obtained.

### Example 4

### Preparation of 3-pyridyl-1-hydroxyethylidene-1, 1-bisphosphonic acid monosodium salt in the presence of recovered olive oil

The procedure of Example I is repeated except recovered olive oil is used as the emulsifying agent instead of sunflower oil. The analysis confirmed the identity of the product and the absence of impurities. Overall yield of 27.0 % is obtained.

### Example 5

### Preparation of 3-pyridyl-1-hydroxyethylidene-1,1-bisphosphonic acid monosodium salt in the presence of paraffin

The procedure of Example I is repeated except paraffin is used as the emulsifying agent instead of sunflower oil. The analysis confirmed the identity of the product and the absence of impurities. Overall yield of 25.0 % is obtained.

### Example 6

### Preparation of 3-pyridyl-1-hydroxyethylidene-1,1-bisphosphonic acid monosodium salt without an emulsifying agent

The procedure of Example I is repeated except any emulsifying agent is used in the reaction medium. As a consequence, it is noticed that the yield of the process relatively lower than the processes wherein disclosed emulsifying agents are employed. Overall yield of 22.0 % is obtained.

### Example 7

### Preparation of 3-pyridyl-1-hydroxyethylidene-1,1-bisphosphonic acid monosodium salt in the presence of nonyl phenol 4 mol ethoxylate

The procedure of Example I is repeated except nonyl phenol 4 mol ethoxylate is used as the emulsifying agent instead of sunflower oil. The analysis confirmed the identity of the product and the absence of impurities. Overall yield of 24.0 % is obtained.

### Example 8

### Preparation of 3-pyridyl-1-hydroxyethylidene-1,1-bisphosphonic acid monosodium salt in the presence of nonyl phenol 6 mol ethoxylate

The procedure of Example I is repeated except nonyl phenol 6 mol ethoxylate is used as the emulsifying agent instead of sunflower oil. The analysis confirmed the identity of the product and the absence of impurities. Overall yield of 23.0 % is obtained.

### Example 9

### Preparation of 3-pyridyl-1-hydroxyethylidene-1,1-bisphosphonic acid monosodium salt in the presence of nonyl phenol 10 mol ethoxylate

The procedure of Example I is repeated except nonyl phenol 10 mol ethoxylate is used as the emulsifying agent instead of sunflower oil. The analysis confirmed the identity of the product and the absence of impurities. Overall yield of 23.5 % is obtained.

## Claims

1. A process for the preparation of 3-pyridyl-1-hydroxyethylidene-1,1-biphosphonic acid or metal salts thereof, comprising the steps of:
(a) reacting 3-pyridyl acetic acid with phosphorous acid and phosphorous trichloride wherein the reaction is carried out in the presence of an emulsifying agent selected from the group consisting of;
(i) aralkyl or alkyl ethoxylates or derivates thereof having the general formula of R-X-O-(CH ₂CH₂O)ₙ-H,
(ii) triglycerides having the general formula of (RCO₂CH₂)₂CHOCOR or oils such as plant and animal oils containing such triglycerides, and
(iii) alkanes having the general formula CₘH₂ₘ₊₂;
where R represents branched or non-branched alkyl groups having 1 to 20 carbons or alkenyl groups having 2 to 20 carbons, n is a number between 1 and 30, X represents phenyl or naphtyl or-CH₂-, and m is a number between 20 and 30.
(b) recovering said 3-pyridyl-1-hydroxyethylidene-1,1-bisphosphonic acid or metal salts thereof by the hydrolysis reaction of phosphorous intermediates.

2. A process according to claim 1 wherein said process further comprises the step of drying crystalline product for obtaining the monohydrate or hemipentahydrate form of 3-pyridyl-1-hydroxyethylidene-1,1-bisphosphonic acid.

3. A process according to claim 1 wherein said metal salt is preferably the sodium salt of 3-pyridyl-1-hydroxyethylidene-1, 1-bisphosphonic acid.

4. A process according to claim 1 wherein the reaction is conducted in the presence of ethoxylates in which R is an alkyl or alkenyl having 4 to 19 carbon atoms, X is phenyl or -CH₂-, and n= 4-30.

5. A process according to claim 1 wherein the reaction is conducted in the presence of ethoxylates in which R is an alkyl or alkenyl group having 9 carbons, X is phenyl and n is a number ranging from 4 to 10.

6. A process according to claim 1 or 5 wherein the reaction is conducted in the presence of ethoxylates selected from the group consisting of nonylphenol 4 mol ethoxylate, nonylphenol 6 mol ethoxylate and nonylphenol 10 mol ethoxylate.

7. A process according to claim 1 wherein the reaction is conducted in the presence of an ethoxylate in which R is an alkenyl group having 11 carbon atoms, X is CH₂ and n=6 wherein the ethoxylate is lauryl alcohol 6 mol ethoxylate.

8. A process according to claim 1 wherein the reaction is conducted in the presence of triglycerides in which R is alkyl or alkenyl group having 12 to 22 carbon atoms and containing one or more double bonds.

9. A process according to claim 1 or 8 wherein the reaction is conducted in the presence of an emulsifying agent selected from the group consisting of sunflower oil, olive oil and corn oil containing said triglycerides.

10. A process according to claim 1 wherein the reaction is conducted in the presence of paraffins in which m is a number ranging from 22 to 27.

11. A process according to claim 1 wherein the reaction is conducted at a temperature ranging from 40 °C to 150 °C.
